Europäisches Patentamt

European Patent Office

Office européen des brevets

(19)

(11) Publication number: **0 211 298**
A2

(12) # EUROPEAN PATENT APPLICATION

(21) Application number: **86109940.6**

(22) Date of filing: **19.07.86**

(51) Int. Cl.⁴: **A 61 K 7/035**
**A 61 K 9/14, C 08 J 9/28**

(30) Priority: **22.07.85 US 757250**

(43) Date of publication of application:
**25.02.87 Bulletin 87/9**

(84) Designated Contracting States:
**BE DE FR GB IT NL SE**

(71) Applicant: DeSoto, Inc.
1700 South Mt. Prospect Road
Des Plaines Illinois 60017(US)

(72) Inventor: Brown, Wallace H.
1303 Gilbert
Downers Grove, III. 60 515(US)

(72) Inventor: Vandeberg, John T.
415 West Oakwood Drive
Barrington, III. 60 010(US)

(72) Inventor: Dachniwskyj, Maryam L.
1320 Cambia Drive 7208
Schaumburg, III. 60193(US)

(72) Inventor: Garlen, David
10 Karen Way
Summit New Jersey 07901(US)

(74) Representative: Reitzner, Bruno, Dr. et al,
Patentanwälte Dipl.-Ing. R. Splanemann Dr. B. Reitzner
Tal 13
D-8000 München 2(DE)

(54) Free-Flowing dry powder compositions containing oily liquid.

(57) Vesiculated beads of cross-linked resin containing an average of at least 5 thin-walled foraminous cells to provide communication between the vesicles within the bead and the exterior of the beads to allow liquid to move into and out of the beads have an emollient incorporated therein. The emollient is preferably a long chain ester or a silicon oil, and the beads containing the emollient are incorporated into dry powder cosmetic compositions, preferably together with talc or clay. The free-flowing dry powder characteristic is provided by the presence of from 2% up to about 20% of finely divided silica.

EP 0 211 298 A2

**0211298**

-1-

# FREE-FLOWING DRY POWDER COMPOSITIONS
## CONTAINING OILY LIQUID
### DESCRIPTION

Technical Field

This invention relates to dry, free-flowing powder compositions, usually for cosmetic application, characterized by the sustained release of a large proportion of emollient or other oily liquid which tends to agglomerate a dry powder composition. It is particularly desired to provide a silky and smooth feeling when applied to skin, and especially to normally dry skin. The invention particularly contemplates face powders, body powders, baby (diaper) powders, and protective hand powders.

Background Art

Cosmetic compositions intended to apply an emollient are in common use, but these compositions are liquids or creams, and not dry powders. As a result, these prior compositions provide an oily appearance, whereas it is desired to give the skin a dry appearance at the same time that the skin is moisturized by the emollient and made to feel smoother and silkier.

It is possible to incorporate emollients into face powders and body powders, but then the powder particles stick together. Liquids providing a water repellent action also tend to agglomerate a powder composition. This makes the powder more difficult to handle and apply, and the desired smooth and silky feel on application is not achieved. Also, very little oil can be held by the powder particles before the powder characteristic is lost.

Another possibility is to encapsulate the oily liquid within some sort of a tiny capsule, but this has presented a very limited choice. While the oil is in the capsule, it is ineffective. After the capsule has been ruptured, the full contents of the capsule become available in one spot, and the emollient sticks the powder particles together at that spot which produces a caked appearance in that area.

In a companion application, it is pointed out that the oily liquid can be incorporated into substantially dry vesiculated beads of cross-linked resin, the beads containing many thin-walled foraminous cells to provide communication between the vesicles within the beads and the exterior of the beads and to allow liquid to seep into and out of the beads. When these beads are loaded with oil, the oil largely remains within the beads when the beads are stored alone or in combination with other powder components, but when these beads are applied to the skin, the oil is slowly released. In this way, the oil is initially present in only small amounts which limits the tendency of the oil to stick the powder particles together, and the oil is constantly renewed on the skin to maintain its presence thereon over an extended period of time.

It is desired in such compositions to have the beads contain a large proportion of emollient or other oily liquid tending to agglomerate the beads, and to limit the proportion of other powder component in the composition. When this is attempted, the emollient or other oily liquid present in the beads tends to agglomerate the powder composition. It is desired to minimize this agglomeration tendency with a minimal addition of other material, especially with respect to the amount of oily liquids which is made available.

-3-

Description of Invention

In accordance with this invention, an emollient or other oily liquid tending to agglomerate a powder composition is incorporated into substantially dry preformed vesiculated beads of cross-linked resin, the beads containing an average of at least 2, more usually at least 5, thin-walled foraminous cells which allow liquid to move into and out of the beads. These cells preferably include at least some cell walls which are discontinuous on electron microscope examination to provide communication between the vesicles within the bead and the exterior of the bead and thus enable movement of liquid by capillary action, though this action may not require openings large enough to be seen. The very thin walls of the cells (about 0.2 micron) are concluded to be foraminous, for water contained therein is easily removed by simple exposure to air even though no fissure large enough for the electron microscope to pick up is found in many of the cells.

It is found that these vesiculated beads can be loaded with oily liquid after they have been formed and dried, and the added oil largely remains within or adsorbed onto the beads to be available for use even when the beads are blended with other powder components. In this way, powder compositions can be formulated with beads containing up to about their own weight of oily liquids.

These oil-containing vesiculated beads in this invention are employed in a dry, free-flowing powder composition by incorporating into the composition which tends to agglomerate, a small proportion of finely divided silica, including hydrated silica, and especially fumed silica. The finely divided silica has been found to be effective

-4-

in amounts of from 2% up to about 20%, preferably from 3% to 10%, based on the total weight of the composition.

One objective of this invention is to incorporate relatively large amounts of oily liquid into a powder composition, and when the oil is merely absorbed onto powder particles, as in the prior art, large amounts of finely divided silica were required to provide the dry, free-flowing characteristic for a powder composition containing relatively little oil. In this invention, the weight ratio of finely divided silica to oily liquid is from 1:5 to 1:20, so much less silica is required to be used with respect to a given quantity of oil than when the oil was absorbed onto powder particles in the prior art.

Another objective of this invention is to provide cosmetic powders which provides a silky and smooth feel when applied to skin. Finely divided silica normally confers a harsh and draggy feel, but the small amounts of silica used herein in comparison with the large amounts of oil in combination with having this oil on a relatively round and smooth light weight polymer bead enables the desired smooth and silky feel to be maintained.

The dry, free-flowing mixture of oil-containing vesiculated beads and fumed silica may be used alone or combined with talc or clay, such as kaolin clay, to provide a powder which can be applied to the skin in various ways. It is preferred that the talc or clay constitute at least about 50% of the weight of the cosmetic composition, but this is not essential.

The beads may contain from about 10% up to about 65%, preferably from 20% to 55% of oily liquid. The preferred oily liquids are emollients, but other liquids tending to cause agglomeration can be used, such a silicone oils which provide a water repellent action. Still other materials may be present in the beads, such as dye solutions, sun blocking agents, medicaments, and perfumes.

These oily liquids are more particularly illustrated by long chain esters, such as isopropyl myristate. Other oily liquids which may be used are illustrated by mineral oil. These oily liquids provide moisturizing compositions. Still other oils which may be used are methyl-terminated polysiloxane oils which provide a water repellent skin protecting powder. Also, the oily liquid may contain all sorts of active agents, such as medicaments or sun blocking agents.

Among the silicone oils, good results have been obtained using dimethicone (a low volatility silicone from General Electric Company SF 96-200 may be used) and cyclomethicone (a high volatility silicone from Union Carbide Corp. #7158 may be used).

The oily liquids which are used herein are drawn into the vesiculated beads after they are formed and dried to remove water therefrom. This is simply carried out by merely mixing the oily liquid and the beads together and holding them together until the beads appear to be relatively dry.

While other components providing special benefit can be incorporated into the powder compositions of this invention, the prime objective of this invention is simply to provide a dry, free-flowing powder composition which can be easily applied to the skin for any of several known purposes. Thus, one can provide a face powder or a body powder which confers a relatively dry surface appearance at the same time that it effectively applies an emollient to confer a smooth and silky feel and to add oil to a skin which may be excessively dry. On the other hand, one may wish to apply a perfume or a sun aborbing agent. In any event, the free-flowing powder characteristic is important to preferred application as is achieved by this invention without regard for the character of the oily liquid or the purpose of the cosmetic.

The vesiculated beads to be useful in this invention must be substantially unground, for aggressive grinding breaks up the beads and impairs their usefulness. The beads may vary in average size of from about 1 micron to about 30 microns, but beads having an average size of from 3 microns to 20 microns are preferred. These beads preferably average at least about 5 vesicles per bead.

The beads in this invention are substantially free from water, and this denotes a water content of less than about 0.2% by weight. The dried beads can be provided by removing water from the water-wet paste of vesiculated beads which is provided when these beads are produced by polymerization in aqueous emulsion.

Water removal can be by simple exposure to air at room temperature, by passing heated air, typically at a temperature of 100°F. to 120°F. over the beads, by tumbling the wet beads in a rotating structure such as a pipe or centrifuge basket while air is passed through the beads, by dropping them through a tower with drying gas moves upwardly therethrough, by vacuum drying, by using an azeotroping solvent, or in any other desired manner.

It should be noted that the beads are usually produced by polymerization in aqueous emulsion so that they are provided in aqueous medium. When this aqueous medium is filtered or allowed to separate with the supernatant aqueous liquor decanted, the the vesiculated beads which are desired are provided in the form of an aqueous cake which includes the surfactants and colloidal material involved in their production. These tend to cause agglomeration when simple drying is used, but this can be avoided by washing the beads with water one or more times to remove these adhesive agents with the wash water. Solvent washing is also permitted, and the water can be azeotropically removed by heating the solvent. Some of the solvent can be allowed to remain to help load the beads with the volatile liquid.

The water-wet paste which is treated in the above manner is formed when excess water is drained from the aqueous suspension obtained when the beads are prepared in aqueous suspension, a surfactant and/or protective colloid being needed to maintain the suspension while the beads are produced. A typical paste is called a cake, and it contains about 65% water while appearing to be dry.

The beads in this invention can be pigmented or unpigmented, depending upon the application which is intended. When pigmentation is desired, the pigment can be incorporated into the walls of the beads as they are formed, and titanium dioxide and iron oxides are typical pigments. One can also associate with the preformed beads a pigment which is incompatible with an azeotropic organic solvent selected to help remove the water by azeotroping it off. Thus, one can add a water-based pigment, such as lamp black, to the aqueous paste either before or after the addition of organic azeotropic solvent to be present while the water is azeotropically removed. This causes the added pigment to adhere to the surface of the beads.

The vesiculated beads are produced in an aqueous suspension which is drained to form a water-wet paste. The procedure for producing these beads involves polymerization in aqueous emulsion in the presence of surfactants and, preferably also, protective colloids. Protective colloids such as polyvinyl alcohol and hydroxyethyl cellulose are frequently used.

The preferred vesiculated beads are styrene-cross-linked unsaturated polyester resins. These are made into a vesiculated bead in conventional fashion, as illustrated by U.S. Pat. No. 3,879,314. Various other patents are of interest to the formation of vesiculated beads useful in this invention, particular attention being directed to U.S. patents Nos. 3,822,224, 3,923,704 and 3,933,579. This last-named patent describes the vesiculated beads which are preferred herein, namely, those having a ratio of granular diameter to mean vesicle diameter of at least 5:1, a vesicle volume of from 5% to 95% of the volume of the granule, and not more than about 60% pigment, by volume.

The vesiculated beads used herein have a highly cross-linked polymeric body which is preferably constituted by a carboxyl-functional unsaturated polyester resin cross-linked with an ethylenically unsaturated monomer copolymerizable therewith. The unsaturation in the polyester is preferably maleate unsaturation, these polyesters being themselves well known and illustrated hereinafter. It is preferred that the polyester have an acid value of 10 to 45 mgm KOH per gm.

During the production process, water migrates into the polymeric beads to swell them, and the polymer in the bead walls polymerizes at the same time. As a result, it is normal to have some of the cell walls fracture, and the extent of fracturing can be controlled by controlling the polymerization process. The more polymerization can be delayed, the greater the number of cell walls which are either not present or disrupted. In this way, the proportion of oily liquid which can be held by the vesiculated beads can be varied.

The unsaturated monomers used for cross-linking are also well known and are water insoluble monomers typically illustrated by styrene or vinyl toluene. The polyesters and monomers which may be used are more fully discussed in U.S. Pat. No. 3,879,314 which shows the production of vesiculated beads using a water-soluble polyamine containing at least three amine groups per molecule and having a dissociation constant in water (pKa value) of 8.5-10.5, typically illustrated by diethylene triamine. The polyamine is used in a concentration providing 0.3 to 1.4 amine groups per polyester carboxyl group. It is preferred to have from 35% to 45% of the unsaturated polyester cross-linked with from 55% to 65% of styrene.

Suitable pigmented vesiculated beads in accordance with this invention are illustrated in U.S. Patent No. 3,879,314 issued April 22, 1975, see particularly Example II. By proceeding in accordance with said Example II and using a polyester of 18% phthalic anhydride, 37% maleic anhydride and 45% propylene glycol dissolved in styrene to form a solution containing 41.8% of the polyester, vesiculated beads pigmented with titanium dioxide, anatase, to contain about 43.2% pigment are

-10-

provided. These beads have an average size of about 9 microns and contain an average of more than 10 cells per bead, and are the beads used in the Examples of this application.

The invention is illustrated in the examples which follow. All parts and proportions herein are by weight unless otherwise specified.

Example 1

An aqueous slurry of vesiculated beads has the bulk of its water content removed by decantation to provide an aqueous paste (termed a beadcake) containing 35 parts of vesicular beads, 64.5 parts water, 0.18 parts of a 75% solution of sodium dioctyl sulfosuccinate (the American Cyanamid surfactant, Aerosol OT may be used), 0.28 parts polyvinyl alcohol and 0.04 parts hydroxy ethyl cellulose. These surfactants and colloids are typical residues of bead production and might cause agglomeration if the water were removed by simple drying. The vesiculated beads are composed of 56.0% resin and 44.0% titanium dioxide. The bead resin is an unsaturated polyester containing propylene glycol/maleic anhydride/phthalic anhydride in proportions of 3.72/2.06/4.22, and this polyester is dissolved in styrene to provide a 58/42 ratio of styrene to polymers. To form the beads, the mixture of polyester and styrene is dispersed in water with the aid of surfactants and protective colloids in the proportions noted and copolymerized in the presence of a quaternary ammonium salt to cause vesicles to form as described in U.S. Pat. No. 3,879,314. The resulting vesiculated beads are in water slurry, and this is drained to provide the beadcake starting material.

The beadcake is then dispersed in 50% of its volume of deionized water with agitation and allowed to settle, the water being decanted to lower the concentration of adhesive agents. This operation is repeated several times with about 300% by volume of deionized water to obtain water-containing beads substantially free of agglomerating adhesive agents. These water-wet beads are dried by spreading them on a tray and passing warm air at a temperature of about 110°F. thereover. The water on and within the beads simply evaporates, and the drying process is quite effective, enabling one to obtain beads which are sufficiently dry to be combined with isocyanate-functional liquids without inducing reaction with water.

Example 2

To a stainless steel kettle equipped with an agitator, add 24.13 pounds of volatile silicone (Union Carbide Corp. product #7158 may be used) and then slowly add 17.31 pounds of the dried beads obtained in Example 1 and mix until a uniform dry crumbly paste is formed. This paste has a "silky" feel and is very smooth. It can be rubbed onto the skin to provide a water repellent emollient effect, but the appearance of the composition is unattractive, it does not sprinkle easily out of a container, and it must be rubbed onto the skin to break up the agglomerates, making application more difficult than it would be if the powder was free-flowing. When 5% of fumed silica is added (the commercial product Cab-O-Sil M5 may be used), and mixed in, the powder becomes free-flowing, and its appearance and application are improved without impairing its silky feel or its capacity to deposit a water repellent emollient film upon the skin. This is surprising because fumed silica normally produces a draggy feel which is not encountered herein.

TABLE I

| Formula No. | 1 | 2 | 3 |
|---|---|---|---|
| Ingredient (% by Wt.) | | | |
| Silicone SF96-200/ White Beads (50/50) | 20.00 | | |
| Silicone SF96-200 | | | 10.00 |
| Light Mineral Oil/ White Beads (50/50) | 20.00 | | |
| Light Mineral Oil | | | 10.00 |
| Fragrance #MF0925/ White Beads (50/50) | 1.00 | | |
| Fragrance #MF0925 | | 0.50 | 0.50 |
| Talc | 54.00 | 94.50 | 74.50 |
| Fumed silica | 5.00 | 5.00 | 5.00 |
| Total. | 100.00 | 100.00 | 100.00 |

Fragrance MF #0925 is a fragrance oil obtainable from V. Mane Fils of Fairfield, NJ.

In formula #2 without beads, the feel and emollient action is absent. In formula #3 without beads, the action of the emollient is apparent, but the powder cakes and does not have a desirable feel. Formula #1 with the oily liquid components taken up within the dried vesiculated beads of Example 1 has a desirable silky feel and results in a soft-feeling skin which has significant water repellency. All of

-13-

this is achieved without an oily appearance or a sticky or draggy feel, and the effect is sustained for several hours.

The silicone oil, mineral oil and fragrance can be combined and incorporated into an equal weight of dry beads to obtain corresponding results.

TABLE II

| Formula No. | 5 | 6 | 7 | 8 |
|---|---|---|---|---|
| Ingredient (% by Wt.) | | | | |
| White Pigmented Beads | 80.00 | 47.50 | 49.50 | 49.50 |
| Silicone SF96-200 | | 47.50 | | 49.50 |
| Mineral Oil | | | 49.50 | |
| Fumed Silica | | 5.00 | 1.00 | 1.00 |
| Isopropyl Myristate | 20.00 | — | — | — |
| Total. | 100.00 | 100.00 | 100.00 | 100.00 |
| Appearance | Lumpy | Fine Powder | Lumpy | Fine Powder |

The function of the fumed silica to provide a free-flowing powder is clear from the tabulated data.

CLAIMS

1. A dry, free-flowing powder composition comprising vesiculated beads of cross-linked resin, said beads containing an average of at least 2 thin-walled foraminous cells to provide communication between the vesicles within said bead and the exterior of said beads to allow liquid to move into and out of said beads, said beads being substantially dry and containing an oily liquid, and there being present in said composition from 2% up to about 20% of finely divided silica to provide the free-flowing powder characteristic.

2. A dry powder as recited in claim 1 in which said silica is a fumed silica.

3. A dry powder as recited in claim 1 in which said silica is present in a weight ratio of finely divided silica to oily liquid of from 1:5 to 1:20.

4. A dry powder as recited in claim 1 in which at least some of said cell walls are discontinuous as determined by electron microscopy, and there are an average of at least 5 thin-walled cells per bead.

5. A dry powder as recited in claim 1 in which said beads are constituted by a carboxyl-functional unsaturated polyester resin cross-linked with an ethylenically unsaturated monomer copolymerizable therewith and have a ratio of granular diameter to mean vesicle diameter of at least 5:1, a vesicle volume of from 5% to 95% of the volume of the granule, and not more than about 60% pigment, by volume.

6. A dry powder as recited in claim 5 in which said polyester resin contains maleate unsaturation and is cross-linked with styrene or

- 2 -

vinyl toluene.

7. A dry powder as recited in claim 1 in which said beads contain from about 10% up to about 65% of said oily liquid based on the weight of the oil-containing beads, and said oily liquid is selected from long chain esters, mineral oil and methyl-terminated polysiloxane oil.

8. A dry powder as recited in claim 1 in which said beads contain from about 20% up to about 55% of said oily liquid based on the weight of the oil-containing beads.

9. A dry powder composition as recited in claim 1 further comprising talc or clay in an amount of at least about 50% of the composition.

10. A dry, free-flowing powder cosmetic composition comprising vesiculated beads of cross-linked resin, said beads containing an average of at least 2 thin-walled foraminous cells to provide communication between the vesicles within said bead and the exterior of said beads to allow liquid to move into and out of said beads, said beads being substantially dry and containing an oily liquid, and there being present in said composition from 2% up to about 20% of fumed silica to provide the free-flowing powder characteristic, said silica being present in a weight ratio of silica to oily liquid of from 1:5 to 1:20.